(19) Europäisches Patentamt

European Patent Office

Office européen des brevets

(11) EP 0 542 645 B1

(12) FASCICULE DE BREVET EUROPEEN

(45) Date de publication et mention
de la délivrance du brevet:
**17.07.1996 Bulletin 1996/29**

(51) Int Cl.6: **C08F 20/06**, C08F 8/44,
C11D 3/37

(21) Numéro de dépôt: **92420403.5**

(22) Date de dépôt: **06.11.1992**

(54) **Polymères et/ou copolymères hydrosolubles à biodégradabilité accrue et leurs applications**

Wasserlösliche Polymere und/oder Copolymere mit erhöhter Biodegradabilität und deren Verwendung

Aqueous soluble polymers and/or copolymers with enhanced biodegradability and their use

(84) Etats contractants désignés:
**AT BE CH DE DK ES GB GR IT LI LU NL PT SE**

(30) Priorité: **12.11.1991 FR 9114141**
**12.11.1991 FR 9114142**

(43) Date de publication de la demande:
**19.05.1993 Bulletin 1993/20**

(73) Titulaire: **COATEX S.A.**
**F-69730 Genay (FR)**

(72) Inventeurs:
• **Egraz, Jean-Bernard**
**F-69130 Ecully (FR)**
• **Ravet, Georges**
**F-69290 Saint-Genis-les-Ollieres (FR)**
• **Rousset, Jacky**
**F-01990 Saint-Trivier-sur-Moignans (FR)**

(56) Documents cités:
DE-A- 3 603 471          FR-M- 3 362
GB-A- 420 533

• CHEMICAL ABSTRACTS, vol. 113, no. 12, 17
Septembre 1990, Columbus, Ohio, US; abstract
no. 99915, 'PREPARATION OF COPOLYMERS
OF ACRYLIC ACID METAL SALTS AND
CYCLOHEXANONE' page 148 & JP-A-02073811

## Description

La présente invention concerne l'augmentation de la biodégradabilité des polymères et/ou copolymères hydrosolubles par leur neutralisation au moyen d'un agent de neutralisation contenant l'ion magnésium ainsi que les produits biodégradables obtenus par ce procédé.

La présente invention concerne également un polymère et/ou copolymère acrylique et/ou vinylique hydrosoluble, biodégradable, utilisé dans des domaines aussi divers que l'industrie du papier, des peintures, des plastiques, du pétrole, des fluides de forage, du traitement d'eau, de la détergence ou encore des cosmétiques, du textile, des encres, du cuir, du broyage et/ou du délitage des minéraux dans l'eau ou autres.

Dans tous ces domaines d'activité, le problème de sauvegarde de l'environnement devient de plus en plus urgent à résoudre. C'est ainsi que, pour des raisons écologiques, l'homme de l'art doit utiliser des composés dont la biodégradabilité est accrue.

Dans ce but, il connaît déjà des polymères biodégradables dont la biodégradabilité résulte d'un greffage d'oxyde de polyalkylène sur des monomères acides tels que l'acide acrylique (EP0429307 et EP0430574), ou de la présence d'anhydride maléique avec des conditions de polymérisation bien définies (EP0396303), ou bien de la terminaison de la chaîne polyacrylate par des groupements hydroxy ou sulfure (US 4095035), ou bien encore par leur composition monomérique (US 4897458 et EP0291808).

Le but de l'invention est de fournir un polymère et/ou copolymère notamment acrylique et/ou vinylique de biodégradabilité accrue, c'est-à-dire dont la dégradation au bout de 36 jours est supérieure à 50% selon la méthode de Sturm modifiée, ceci afin de correspondre au mieux à la réglementation relative à la protection de l'environnement (Journal Officiel de la C.E. n° L251).

Un autre but de l'invention est de fournir un polymère et/ou copolymère acrylique et/ou vinylique non réticulé de biodégradabilité accrue, de viscosité spécifique au plus égale à 25, et de préférence au plus égale à 10.

Enfin un autre but de l'invention est de proposer un procédé comportant une étape de neutralisation totale ou partielle des sites acides actifs des polymères et/ou copolymères acryliques et/ou vinyliques par un agent de neutralisation contenant l'ion magnésium en vue de conférer à ces dits polymères une biodégradabilité supérieure à 50 % au bout de 36 jours selon le test de Sturm modifié.

De manière surprenante, la Demanderesse a constaté que le but de l'invention est obtenu par l'utilisation comme agent de neutralisation d'un agent contenant l'ion magnésium et plus particulièrement lorsque 35 à 90% de préférence 35 à 60% des sites acides actifs du polymère et/ou copolymère acrylique et/ou vinylique est neutralisé par l'ion magnésium; en outre de l'agent contenant l'ion magnésium au moins un agent de neutralisation disposant d'un ion monovalent pouvant neutraliser jusqu'à 65 %, ou bien 40 % des sites acides actifs peut être utilisé.

Ces polymères et/ou copolymères acryliques et/ou vinyliques sont obtenus selon des procédés connus de la polymérisation radicalaire, en présence de régulateurs de polymérisation tels que, par exemple, des composés organiques à base d'hydroxylamine et en présence d'initiateurs de polymérisation tels que les peroxydes et les persels, par exemple l'eau oxygénée, le persulfate, l'hypophosphite de sodium, l'acide hypophosphoreux, de l'un au moins des monomères et/ou comonomères suivants : acide acrylique et/ou méthacrylique, itaconique, crotonique, fumarique, anhydride maléique, ou encore isocrotonique, aconitique, mésaconique, sinapique, undécylénique, angélique, canellique, hydroxyacrylique, sous forme acide ou partiellement neutralisée, l'acroléine, l'acrylamide, l'acrylonitrile, les esters des acides acrylique et méthacrylique, et en particulier le méthacrylate de diméthylaminoéthyle, vinylpyrrolidone, vinylcaprolactame, l'éthylène, le propylène, l'isobutylène, le diisobutylène, l'acétate de vinyle, le styrène, l'alphaméthylstyrène, la méthylvinylcétone, dans un milieu de polymérisation qui peut être l'eau, le méthanol, l'éthanol, le propanol, l'isopropanol, les butanols, ou leurs mélanges, ou encore le diméthylformamide, le diméthylsulfoxyde, le tétrahydrofurane, l'acétone, la méthyléthylcétone, l'acétate d'éthyle, l'acétate de butyle, l'hexane, l'heptane, le benzène, le toluène, le xylène, le mercaptoéthanol, le tertiododécylmercaptan, l'acide thioglycolique et ses esters, le n-dodécylmercaptan, les acides acétique, tartrique, lactique, citrique, gluconique, glucoheptonique, l'acide 2-mercaptopropionique, le thiodiéthanol, les solvants halogénés comme le tétrachlorure de carbone, le chloroforme, le chlorure de méthylène, les éthers de monopropylèneglycol, diéthylèneglycol ou leur mélange.

La solution du polymérisat acide obtenue est alors partiellement ou totalement neutralisée à raison de 35 à 90% d'un agent de neutralisation contenant l'ion magnésium, et éventuellement d'un agent de neutralisation contenant un ion monovalent.

L'agent de neutralisation monovalent est choisi dans le groupe constitué par les cations alcalins (tels que le sodium, le lithium et le potassium), l'ammonium, les amines primaires, secondaires ou tertiaires aliphatiques et/ou cycliques (telles que, par exemple, les éthanolamines (mono, di, tri-éthanolamine), la mono et diéthylamine, la cyclohexylamine, la methylcyclohexylamine).

La solution du polymérisat acide ou ainsi neutralisé peut également être traitée selon des procédés statiques ou dynamiques connus de l'homme de l'art par un ou plusieurs solvants polaires appartenant notamment au groupe constitué par le méthanol, l'éthanol, le propanol, l'isopropanol, les butanols, l'acétone, le tétrahydrofurane, produisant ainsi une séparation en deux phases, chacune récupérée séparément.

La phase la moins dense comporte la majeure fraction du solvant polaire et constitue la fraction de polymère et/ou copolymère de bas poids moléculaire, tandis que la phase aqueuse la plus dense constitue la fraction des polymères et/ou copolymères de poids moléculaire plus élevé.

Les polymères et/ou copolymères selon l'invention ont généralement une viscosité spécifique au plus égale à 25 et de préférence au plus égale à 10.

Cette viscosité spécifique des polymères et/ou copolymères est symbolisée par la lettre "η" et est déterminée de la manière suivante.

On prend une solution du polymérisat de façon à obtenir une solution correspondant à 2,5 g de polymère sec et à 50 ml d'une solution de chlorure de sodium à 60 g/l.

Puis, on mesure avec un viscosimètre capillaire de constante de Baume égale à 0,000105 placé dans un bain thermostaté à 25°C le temps d'écoulement d'un volume donné de la solution précitée contenant le polymère et/ou copolymère, ainsi que le temps d'écoulement du même volume de solution aqueuse de chlorure de sodium dépourvue dudit polymère et/ou copolymère. Il est alors possible de définir la viscosité "η" grâce à la relation suivante :

$$\eta = \frac{\text{(temps d'écoulement de la solution de polymère)} - \text{(temps d'écoulement de la solution NaCl)}}{\text{temps d'écoulement de la solution NaCl}}$$

Le tube capillaire est généralement choisi de telle manière que le temps d'écoulement de la solution de NaCl, dépourvue de polymère et/ou copolymère, soit d'environ 90 à 100 secondes, donnant ainsi des mesures de viscosité spécifique d'une très bonne précision.

Ces polymères et/ou copolymères acryliques et/ou vinyliques selon l'invention peuvent également être traités par tout moyen connu pour les isoler sous la forme d'une fine poudre.

En pratique, la mesure de la biodégradabilité s'effectue à l'aide du test de Sturm modifié, décrit dans le Journal Officiel de la Communauté Européenne n° L251 du 19.09.1984, et dans les Lignes Directrices de l'O.C.D.E. (Organisation de Coopération et de Développement Economiques) n°301B du 12.05.1981. Cette biodégradabilité, exprimée en pourcentage, représente en fait la quantité de $CO_2$ produit sur une période de 36 jours que cette substance est capable de produire, calculée sur la base de sa teneur en carbone. Avant de procéder à ces mesures, il est nécessaire de préparer les différents constituants du test qui sont :

- une solution d'hydroxyde de baryum ($Ba(OH)_2$ - $8H_2O$) afin de piéger et titrer la quantité de $CO_2$ produite pendant la durée de l'essai,
- un inoculum servant à inoculer les microorganismes provenant de boues activées et contrôlées par substance de référence, en l'occurence une solution d'acétate de sodium à 20 mg/l,
- l'échantillon de la substance à tester ainsi que le milieu pour le test.

En effet, ce milieu est constitué de solutions de divers réactifs, dites solutions stocks, obtenues à partir d'eau de haute qualité, c'est-à-dire d'eau distillée ne contenant pas de substance toxique (notamment du cuivre), avec une faible teneur en carbone (0,5 mg TOC/l) (TOC = Total Organic Carbon) et une résistivité supérieure ou égale à 18 M /cm, et se compose par litre d'eau de haute qualité de :

- 4 ml d'une solution de chlorure ferrique obtenue par dissolution de 0,25g de $FeCl_3$ - $6H_2O$ dans un litre d'eau de haute qualité,
- 1 ml d'une solution de sulfate de magnésium obtenue par dissolution de 22,50 g de $MgSO_4$ - $7H_2O$ dans un litre d'eau de haute qualité,
- 1 ml d'une solution de chlorure de calcium obtenue par dissolution de 27,50 g de $CaCl_2$ anhydre dans un litre d'eau de haute qualité,
- 2 ml d'une solution de tampon phosphate obtenue par dissolution de 8,50 g de dihydrogénophosphate de potassium ($KH_2PO_4$), 21,75 g d'hydrogénophosphate de dipotassium ($K_2HPO_4$), 33,40 g de dihydrate hydrogénophosphate de dissodium ($Na_2HPO_4$ - $2H_2O$) et de 1,70 g de chlorure d'ammonium ($NH_4Cl$) dans un litre d'eau de haute qualité
- 1 ml d'une solution de sulfate d'ammonium obtenue par dissolution de 40g de $(NH_4)_2SO_4$ dans un litre d'eau de haute qualité.

Ces dissolutions étant effectuées pour 3 litres de milieu réactionnel, il est nécessaire de préparer l'inoculum. L'inoculum utilisé provient de boues activées fraîchement prélevées dans une station d'épuration d'eaux d'égouts en bon ordre de marche. Cette station ne doit pas traiter d'effluents industriels, si ce n'est en quantités négligeables (environ 10%).

A leur arrivée au laboratoire, les boues activées sont aérées pendant quatre heures, puis on prélève 500 ml de la suspension et on homogénéise pendant deux minutes à vitesse moyenne à l'aide d'un agitateur magnétique avant de décanter pendant 1/2 heure.

Si le surnageant contient toujours une quantité importante de particules solides de boue au bout de 30 minutes, on peut, soit le laisser encore décanter pendant 30 à 60 minutes, soit le traiter de façon à obtenir une meilleure décantation.

Il est nécessaire de laisser décanter le surnageant de manière à disposer d'un volume suffisant pour ensemencer un inoculum à 1% par fiole de détermination de $CO_2$, et d'éviter d'entraîner des particules solides de boue qui risqueraient de fausser la mesure de la production de $CO_2$.

Il est inutile d'effectuer des comptages dans la fraction surnageante afin de déterminer le nombre de micro-organismes. L'inoculum doit normalement contenir de $10^6$ à $20 \times 10^6$ unités ou colonies par ml. Il doit être utilisé le jour même de sa préparation.

Tout ceci fait, il suffit d'introduire dans 3 bonbonnes différentes un témoin sans substance à étudier, une concentration de substance à étudier (10 mg/l) et une référence (acétate de sodium) dont la biodégradabilité doit être supérieure à 60%, 2470 ml d'eau de haute qualité ainsi que chacune des solutions stocks en quantité correspondant à 3 litres d'eau de haute qualité et 30 ml d'inoculum, puis aérer le mélange par un bullage d'air exempt de $CO_2$ (après passage dans un bain d'hydroxyde de baryum) pendant 24 heures avant le début du test.

La solution d'hydroxyde de baryum est passée sur papier filtre et conservée à l'abri de l'air afin de prévenir toute absorption de $CO_2$.

Le test commence après 24 heures de bullage par l'introduction dans chacune des bonbonnes contenant le mélange aéré :

- de la substance à analyser à une concentration de 10 mg/l,
- de la substance de référence à une concentration de 20 mg/l,
- de rien afin de constituer un blanc.

Le $CO_2$ produit dans chaque bonbonne réagit avec l'hydroxyde de baryum; on détermine la quantité de $CO_2$ produite par titrage du $Ba(OH)_2$ restant au moyen de HCL 0,05 N. A cet effet, on débranche périodiquement (tous les deux ou trois jours) le flacon absorbeur de $CO_2$ le plus proche de la bonbonne.

Après avoir retiré les flacons les plus proches des bonbonnes, on titre la solution de $Ba(OH)_2$ à l'aide de HCL 0,05 N en utilisant de la phénolphtaléine comme indicateur.

On rapproche ensuite de la bonbonne les deux flacons absorbeurs restants en les décalant chacun d'une place, et on ajoute après chaque dosage en fin de série un nouveau flacon absorbeur contenant 100ml d'une solution fraîche de $Ba(OH)_2$ à 0,025 N. On effectue les titrages quand cela est nécessaire (avant l'apparition d'un précipité de $BaCO_3$ dans le deuxième piège après celui de NaOH concentrée à 8 N), soit environ tous les deux jours pendant les dix premiers jours, puis tous les cinq jours jusqu'au 36ème jour. L'arrêt de l'expérimentation est fonction de l'apparition du plateau traduisant la dégradation de la substance à étudier.

Le 35ème jour, on mesure à nouveau le pH des solutions contenues dans les bonbonnes et on ajoute 1 ml de HCL concentré dans chaque bonbonne, afin d'éliminer le carbonate inorganique. On aère les bonbonnes pendant la nuit et on prélève des échantillons dans chacune d'elles pour déterminer le carbone organique dissous (COD), qui n'intervient qu'à titre d'information sur l'ensemble de la période écoulée, et on effectue le titrage le 36ème jour.

La portée et l'intérêt de l'invention seront mieux perçus grâce aux exemples suivants.

## **Exemple 1**

Cet exemple concerne la mesure de biodégradabilité du même acide polyacrylique neutralisé par différents ions.

L'essai n°1 illustrant l'art antérieur indique la biodégradabilité d'un polyacrylate de sodium neutralisé à 100% par du sodium et de viscosité spécifique 0,54.

L'essai n°2 illustrant l'invention indique la biodégradabilité d'un polyacrylate mixte de sodium et de magnésium obtenu par neutralisation totale, de telle sorte que 50% des sites acides actifs sont neutralisés par le sodium et 50% par le magnésium et de viscosité spécifique 0,54.

L'essai n°3 illustrant l'invention indique la biodégradabilité d'un polyacrylate mixte de sodium et de magnésium obtenu par neutralisation totale de telle sorte que 25% des sites acides actifs sont neutralisés par le sodium et 75% par le magnésium et de viscosité spécifique 0,54.

Pour tous ces essais, la source de micro-organismes provient de boues activées prélevées à la station d'épuration de Pierre Bénite (France), et les conditions opératoires sont celles précitées.

Tous les résultats sont consignés dans le tableau 1.

## TABLEAU 1

| ESSAI No. | | Neutralisation | | Nombre de jours | Biodégradabilité % |
| --- | --- | --- | --- | --- | --- |
| | | Taux de groupes acides neutralisés | Cation neutralisant | | |
| 1 | Art antérieur | 100 | Na | 36 | 43 |
| 2 | Invention | 50/50 | Na/Mg | 36 | 54 |
| 3 | Invention | 25/75 | Na/Mg | 36 | 65 |

La lecture du tableau 1 permet de constater la différence de biodégradabilité entre le polymère de l'art antérieur et celui selon l'invention.

En effet, pour l' essai n°1, le pourcentage de la quantité théorique de $CO_2$ est de 43% après 36 jours, alors que celui de l'essai n°2 est de 54% après 36 jours et celui de l'essai n°3 est de 65% après 36 jours.

Ainsi, seuls les polymères selon l'invention obtenus par la neutralisation d'un acide polyacrylique au moyen d'un mélange neutralisant contenant l'ion magnésium donnent une biodégradabilité supérieure à 50% au bout de 36 jours de test.

**Exemple 2**

Cet exemple concerne la mesure de biodégradabilité du même acide polyacrylique de départ, de viscosité spécifique 0,54 neutralisé avec des taux de magnésium allant jusqu'à 88% des sites acides actifs du polymère.

L'essai n°4 mesure la biodégradabilité d'un polyacrylate mixte de sodium et de magnésium neutralisé de telle sorte que 70% des sites acides actifs sont neutralisés par le sodium et 30% par le magnésium.

L'essai n°5 mesure la biodégradabilité d'un polyacrylate mixte de sodium et de magnésium neutralisé de telle sorte que 60% des sites acides actifs sont neutralisés par le sodium et 40% par le magnésium.

L'essai n°6 mesure la biodégradabilité d'un polyacrylate mixte de sodium et de magnésium neutralisé de telle sorte que 50% des sites acides actifs sont neutralisés par le sodium et 50% par le magnésium.

L'essai n°7 mesure la biodégradabilité d'un polyacrylate mixte de sodium et de magnésium neutralisé de telle sorte que 35% des sites acides actifs sont neutralisés par le sodium et 65% par le magnésium.

L'essai n°8 mesure la biodégradabilité d'un polyacrylate mixte de sodium et de magnésium neutralisé de telle sorte que 25% des sites acides actifs sont neutralisés par le sodium et 75% par le magnésium.

L'essai n°9 mesure la biodégradabilité d'un polyacrylate mixte de sodium et de magnésium neutralisé de telle sorte que 12% des sites acides actifs sont neutralisés par le sodium et 88% par le magnésium.

L'essai n°10 mesure la biodégradabilité d'un polyacrylate dont 50% des sites acides actifs sont neutralisés par l'ion magnésium.

L'essai n°11 mesure la biodégradabilité d'un polyacrylate de même viscosité spécifique 0,54, dont 75% des sites acides actifs sont neutralisés par l'ion magnésium.

La source de micro-organismes et les conditions opératoires du test sont identiques à celles de l'exemple 1.

Tous les résultats sont consignés dans le tableau 2.

TABLEAU 2

| ESSAI No. | Neutralisation | | Nombre de jours | Biodégradabilité % |
|---|---|---|---|---|
| | Taux de groupes acides neutralisés | Cation neutralisant | | |
| 4 | 70/30 | Na/Mg | 36 | 44 |
| 5 | 60/40 | Na/Mg | 36 | 51 |
| 6 | 50/50 | Na/Mg | 36 | 54 |
| 7 | 35/65 | Na/Mg | 36 | 57 |
| 8 | 25/75 | Na/Mg | 36 | 65 |
| 9 | 12/88 | Na/Mg | 36 | 71 |
| 10 | 50 | Mg | 36 | 52 |
| 11 | 75 | Mg | 36 | 63 |

La lecture des résultats consignés dans le tableau 2 permet de constater que, pour les essais n°5 à n°11, le pourcentage de la quantité de $CO_2$ théorique est supérieur à 50% alors que, pour l'essai n°4, il n'est que de 44%.

Ainsi, un taux de neutralisation par l'ion Mg allant de 35 à 90% des sites acides actifs permet d'obtenir un polymère, selon l'invention, ayant une biodégradabilité améliorée supérieure à 50% après 36 jours de test. Les meilleurs résultats sont obtenus pour un polymère selon l'invention, dont 75% des sites acides actifs sont neutralisés par l'ion magnésium et 25% par l'ion sodium et mieux encore lorsque 88% des sites acides actifs sont neutralisés par l'ion magnésium et 12% par l'ion sodium.

**Exemple 3**

Cet exemple concerne la mesure de la biodégradabilité d'acides polyacryliques de poids moléculaires différents.

L'essai n°12 mesure la biodégradabilité d'un polyacrylate mixte de sodium et de magnésium obtenu par neutralisation totale de telle sorte que 50% des sites acides actifs sont neutralisés par l'ion sodium et 50% par l'ion magnésium, et de viscosité spécifique 0,3.

L'essai n°13 est identique à l'essai n°2 (viscosité spécifique 0,54).

L'essai n° 14 mesure la biodégradabilité d'un polyacrylate mixte de sodium et de magnésium obtenu par neutralisation totale de telle sorte que 50% des sites acides actifs sont neutralisés par l'ion sodium et 50% par l'ion magnésium, et de viscosité spécifique 5,0.

L'essai n°15 mesure la biodégradabilité d'un polyacrylate mixte de sodium et de magnésium obtenu par neutralisation totale de telle sorte que 25% des sites acides actifs sont neutralisés par l'ion sodium et 75% par l'ion magnésium, et de viscosité spécifique 0,3.

L'essai n°16 est identique à l'essai n°3 (viscosité spécifique 0,54).

L'essai n°17 mesure la biodégradabilité d'un polyacrylate mixte de sodium et de magnésium obtenu par neutralisation totale de telle sorte que 25% des sites acides actifs sont neutralisés par l'ion sodium et 75% par l'ion magnésium, et de viscosité spécifique 5,0.

La source de micro-organismes et les conditions opératoires du test sont identiques à celles de l'exemple 1.

Tous les résultats sont consignés dans le tableau 3.

TABLEAU 3

| ESSAI No | Neutralisation | | Viscosité spécifique | Nombre de jours | Biodégradabilité % |
|---|---|---|---|---|---|
| | Taux de groupes acides neutralisés | Cation neutralisant | | | |
| 12 | 50/50 | Na/Mg | 0,3 | 36 | 57 |
| 13 | 50/50 | Na/Mg | 0,54 | 36 | 54 |
| 14 | 50/50 | Na/Mg | 5,0 | 36 | 51 |
| 15 | 25/75 | Na/Mg | 0,3 | 36 | 77 |
| 16 | 25/75 | Na/Mg | 0,54 | 36 | 65 |
| 17 | 25/75 | Na/Mg | 5,0 | 36 | 59 |

La lecture des résultats consignés dans le tableau 3 permet de constater que, quelle que soit la viscosité spécifique, c'est-à-dire le poids moléculaire du polymère, les polymères selon l'invention, neutralisés en partie par l'ion magnésium, ont une biodégradabilité améliorée supérieure à 50% après 36 jours de test.

## Exemple 4

Cet exemple concerne la mesure de la biodégradabilité d'autres polymères neutralisés ou non par l'ion magnésium.

L'essai n°18 mesure la biodégradabilité d'un polymère obtenu par polymérisation et greffage d'acide acrylique sur un polyéthylène glycol de poids moléculaire 3400 et neutralisé à 100% par du sodium, tel que décrit dans l'exemple 1 du brevet EP0429307.

L'essai n°19 mesure la biodégradabilité du même polymère que celui de l'essai n°18, mais neutralisé à 100% par un mélange correspondant à une neutralisation de 50% sodium et 50% magnésium.

L'essai n°20 mesure la biodégradabilité du même polymère que celui de l'essai n°18, mais neutralisé à 100% par un mélange correspondant à une neutralisation de 25% sodium et de 75% magnésium.

La source de micro-organismes et les conditions opératoires du test sont identiques à celles de l'exemple 1.

Tous les résultats sont consignés dans le tableau 4.

## TABLEAU 4

| ESSAI No. | | Neutralisation | | Nombre de jours | Biodégradabilité % |
|---|---|---|---|---|---|
| | | Taux de groupes acides neutralisés | Cation neutralisant | | |
| 18 | Art antérieur | 100 | Na | 36 | 48 |
| 19 | Invention | 50/50 | Na/Mg | 36 | 61 |
| 20 | Invention | 25/75 | Na/Mg | 36 | 68 |

La lecture des résultats consignés dans le tableau 4 permet de constater la différence de biodégradabilité entre le polymère de l'art antérieur et ceux selon l'invention.

En effet, pour l'essai n°18, la biodégradabilité est de 48% après 36 jours de test, alors que celui de l'essai n°19 est de 61% après 36 jours de test et celui de l'essai n°20 est de 68% après 36 jours de test.

Ainsi, l'utilisation selon l'invention d'un agent de neutralisation contenant l'ion magnésium donne des polymères à biodégradabilité améliorée supérieure à 50% après 36 jours de test.

**Revendications**

1. Polymère et/ou copolymère acrylique et/ou vinylique non réticulé, hydrosoluble biodégradable résultant, soit de la polymérisation et/ou copolymérisation de monomères acryliques et/ou vinyliques, soit du traitement par un ou plusieurs solvants polaires selon des procédés statiques ou dynamiques de polymères et/ou copolymères acryliques et/ou vinyliques, soit du greffage de monomères acryliques et/ou vinyliques sur des substrats à base de polyalkylène glycol, caractérisé en ce que les sites acides actifs dudit polymère et/ou copolymère sont totalement ou partiellement neutralisés par au moins un agent de neutralisation contenant l'ion magnésium, de telle sorte que le pourcentage de neutralisation des sites acides actifs par l'ion magnésium varie entre 35% et 90% et, de plus, éventuellement par au moins un agent de neutralisation disposant d'un ion monovalent pouvant neutraliser jusqu'à 65% des sites acides actifs, et en ce que la biodégradabilité est supérieure à 50% au bout de 36 jours selon le test de Sturm modifié.

2. Polymère et/ou copolymère acrylique et/ou vinylique hydrosoluble, biodégradable selon la revendication 1 caractérisé en ce que les sites acides actifs dudit polymère et/ou copolymère sont totalement ou partiellement neutralisés par au moins un agent de neutralisation contenant l'ion magnésium, de telle sorte que le pourcentage de neutralisation des sites acides actifs par l'ion magnésium varie entre 35% et 60% et, de plus, éventuellement par au moins un agent de neutralisation disposant d'un ion monovalent pouvant neutraliser jusqu'à 65% des sites acides actifs, et en ce que la biodégradabilité est supérieure à 50% au bout de 36 jours selon le test de Sturm modifié.

3. Polymère et/ou copolymère acrylique et/ou vinylique hydrosoluble, biodégradable selon la revendication 1 caractérisé en ce que les sites acides actifs dudit polymère et/ou copolymère sont totalement ou partiellement neutralisés par au moins un agent de neutralisation contenant l'ion magnésium, de telle sorte que le pourcentage de neutralisation des sites acides actifs par l'ion magnésium varie entre 60% et 90% et, de plus, éventuellement par au moins un agent de neutralisation disposant d'un ion monovalent pouvant neutraliser jusqu'à 40% des sites acides actifs, et en ce que la biodégradabilité est supérieure à 50% au bout de 36 jours selon le test de Sturm modifié.

4. Polymère et/ou copolymère acrylique et/ou vinylique hydrosoluble biodégradable selon l'une des revendications 1 à 3, caractérisé en ce que l'ion monovalent de l'agent de neutralisation est choisi parmi les ions sodium, potassium, lithium, ammonium ou bien les amines primaires, secondaires ou tertiaires aliphatiques et/ou cycliques.

5. Polymère et/ou copolymère acrylique et/ou vinylique hydrosoluble biodégradable selon l'une des revendications 1, 2 ou 4, caractérisé en ce que 50% des sites acides actifs dudit polymère et/ou copolymère sont neutralisés par un agent de neutralisation contenant l'ion magnésium, et 50% par un agent de neutralisation contenant l'ion sodium.

6. Polymère et/ou copolymère acrylique et/ou vinylique hydrosoluble biodégradable selon l'une des revendications 1, 3 ou 4 caractérisé en ce que 75% des sites acides actifs dudit polymère et/ou copolymère sont neutralisés par un agent de neutralisation contenant l'ion magnésium et 25% par un agent de neutralisation contenant l'ion sodium.

7. Polymère et/ou copolymère acrylique et/ou vinylique hydrosoluble biodégradable selon l'une des revendications 1, 3 ou 4 caractérisé en ce que 88% des sites acides actifs dudit polymère et/ou copolymère sont neutralisés par un agent de neutralisation contenant l'ion magnésium et 12% par un agent de neutralisation contenant l'ion sodium.

8. Polymère et/ou copolymère acrylique et/ou vinylique hydrosoluble biodégradable selon l'une quelconque des revendications 1 à 7, caractérisé en ce qu'il a une viscosité spécifique au plus égale à 25, et de préférence au plus égale à 10.

9. Polymère et/ou copolymère acrylique et/ou vinylique hydrosoluble biodégradable selon la revendication 8, caractérisé en ce qu'il est une solution.

10. Polymère et/ou copolymère acrylique et/ou vinylique hydrosoluble biodégradable selon la revendication 8, caractérisé en ce qu'il est une poudre.

11. Application du polymère et/ou copolymère acrylique et/ou vinylique hydrosoluble biodégradable selon les revendications 1 à 10 aux domaines du papier, des peintures, des plastiques, du pétrole, des fluides de forage, du traitement d'eau, de la détergence, de la cosmétique, du textile, des encres, du cuir, du broyage et/ou du délitage des minéraux dans l'eau.

12. Procédé pour conférer à des polymères et/ou copolymères hydrosolubles une biodégradabilité accrue supérieure à 50% au bout de 36 jours selon le test de Sturm modifié caractérisé en ce qu'il comporte une étape de neutralisation totale ou partielle des sites acides actifs desdits polymères et/ou copolymères par un agent contenant l'ion magnésium.

13. Polymère et/ou copolymère hydrosoluble à biodégradabilité supérieure à 50 % au bout de 36 jours selon le test de Sturm modifié obtenu par le procédé selon la revendication 12.

**Patentansprüche**

1. Wasserlösliches, biologisch abbaubares, unvernetztes acrylisches und/oder vinylisches Polymer und/oder Copolymer, das entweder aus der Polymerisation und/oder Copolymerisation von acrylischen und/oder vinylischen Monomeren oder aus der Behandlung von acrylischen und/oder vinylischen Polymeren und/oder Copolymeren mit einem oder mehreren polaren Lösungsmitteln gemäß statischen oder dynamischen Verfahren oder aus der Pfropfung von acrylischen und/oder vinylischen Monomeren auf Substrate auf der Basis von Polyalkylenglycol hervorgeht, dadurch gekennzeichnet, daß die aktiven sauren Stellen des Polymers und/oder Copolymers ganz oder teilweise neutralisiert sind durch mindestens ein Neutralisationsmittel, das das Magnesiumion enthält, derart, daß der Prozentsatz der Neutralisation der aktiven sauren Stellen durch das Magnesiumion zwischen 35% und 90% variiert, und darüber hinaus gegebenenfalls durch mindestens ein Neutralisationsmittel, das über ein einwertiges Ion verfügt, das bis zu 65% der aktiven sauren Stellen neutralisieren kann, und dadurch, daß die biologische Abbaubarkeit nach 36 Tagen mehr als 50% gemäß dem modifizierten Sturm-Test beträgt.

2. Wasserlösliches, biologisch abbaubares acrylisches und/oder vinylisches Polymer und/oder Copolymer nach Anspruch 1, dadurch gekennzeichnet, daß die aktiven sauren Stellen des Polymers und/oder Copolymers ganz oder teilweise neutralisiert sind durch mindestens ein Neutralisationsmittel, das das Magnesiumion enthält, derart, daß der Prozentsatz der Neutralisation der aktiven sauren Stellen durch das Magnesiumion zwischen 35% und 60% variiert, und darüber hinaus gegebenenfalls durch mindestens ein Neutralisationsmittel, das über ein einwertiges Ion verfügt, daß bis zu 65% der aktiven sauren Stellen neutralisieren kann, und dadurch, daß die biologische Abbaubarkeit nach 36 Tagen mehr als 50% gemäß dem modifizierten Sturm-Test beträgt.

3. Wasserlösliches, biologisch abbaubares acrylisches und/oder vinylisches Polymer und/oder Copolymer nach Anspruch 1, dadurch gekennzeichnet, daß die aktiven sauren Stellen des Polymers und/oder Copolymers ganz oder teilweise neutralisiert sind durch mindestens ein Neutralisationsmittel, das das Magnesiumion enthält, derart, daß der Prozentsatz der Neutralisation der aktiven sauren Stellen durch das Magnesiumion zwischen 60% und 90% variiert, und darüber hinaus gegebenenfalls durch mindestens ein Neutralisationsmittel, das über ein einwertiges Ion verfügt, das bis zu 40% der aktiven sauren Stellen neutralisieren kann, und dadurch, daß die biologische Abbaubarkeit nach 36 Tagen mehr als 50% gemäß dem modifizierten Sturm-Test beträgt.

4. Wasserlösliches, biologisch abbaubares acrylisches und/oder vinylisches Polymer und/oder Copolymer nach einem der Ansprüche 1 bis 3, dadurch gekennzeichnet, daß das einwertige Ion des Neutralisationsmittels aus den Natrium-, Kalium-, Lithium-, Ammoniumionen oder aus den primären, sekundären oder tertiären aliphatischen und/oder cyclischen Aminen ausgewählt ist.

5. Wasserlösliches, biologisch abbaubares acrylisches und/oder vinylisches Polymer und/oder Copolymer nach einem der Ansprüche 1, 2 oder 4, dadurch gekennzeichnet, daß 50% der aktiven sauren Stellen des Polymers und/oder Copolymers durch ein Neutralisationsmittel, das das Magnesiumion enthält, und 50% durch ein Neutralisationsmittel, das das Natriumion enthält, neutralisiert sind.

6. Wasserlösliches, biologisch abbaubares acrylisches und/oder vinylisches Polymer und/oder Copolymer nach einem der Ansprüche 1, 3 oder 4, dadurch gekennzeichnet, daß 75% der aktiven sauren Stellen des Polymers und/oder Copolymers durch ein Neutralisationsmittel, das das Magnesiumion enthält, und 25% durch ein Neutralisationsmittel, das das Natriumion enthält, neutralisiert sind.

7. Wasserlösliches, biologisch abbaubares acrylisches und/oder vinylisches Polymer und/oder Copolymer nach einem der Ansprüche 1, 3 oder 4, dadurch gekennzeichnet, daß 88% der aktiven sauren Stellen des Polymers und/oder Copolymers durch ein Neutralisationsmittel, das das Magnesiumion enthält, und 12% durch ein Neutralisationsmittel, das das Natriumion enthält, neutralisiert sind.

8. Wasserlösliches, biologisch abbaubares acrylisches und/oder vinylisches Polymer und/oder Copolymer nach irgendeinem der Ansprüche 1 bis 7, dadurch gekennzeichnet, daß es eine spezifische Viskosität von höchstens 25 und vorzugsweise von höchstens 10 aufweist.

9. Wasserlösliches, biologisch abbaubares acrylisches und/oder vinylisches Polymer und/oder Copolymer nach Anspruch 8, dadurch gekennzeichnet, daß es sich um eine Lösung handelt.

10. Wasserlösliches, biologisch abbaubares acrylisches und/oder vinylisches Polymer und/oder Copolymer nach Anspruch 8, dadurch gekennzeichnet, daß es sich um ein Pulver handelt.

11. Verwendung des wasserlöslichen, biologisch abbaubaren acrylischen und/oder vinylischen Polymers und/oder Copolymers nach den Ansprüchen 1 bis 10 auf den Gebieten des Papiers, der Anstrichfarben, der Kunststoffe, des Erdöls, der Fluide zum Bohren, der Behandlung von Wasser, der Reinigung, der Kosmetik, der Textilien, der Farben, des Leders, des Mahlens und/oder des Dispergierens von Mineralien in Wasser.

12. Verfahren, bei dem man wasserlöslichen Polymeren und/oder Copolymeren eine erhöhte biologische Abbaubarkeit nach 36 Tagen von mehr als 50% gemäß dem modifizierten Sturm-Test verleiht, dadurch gekennzeichnet, daß es eine Stufe der vollständigen oder teilweisen Neutralisation der aktiven sauren Stellen der Polymere und/oder Copolymere durch ein Mittel umfaßt, welches das Magnesiumion enthält.

13. Wasserlösliches Polymer und/oder Copolymer mit einer biologischen Abbaubarkeit nach 36 Tagen von mehr als 50% gemäß dem modifizierten Sturm-Test, welches durch das Verfahren nach Anspruch 12 erhalten wird.


## Claims

1. Water-soluble, bio-degradable, non-cross-linked acrylic and/or vinyl polymer and/or copolymer produced either by the polymerisation and/or copolymerization of acrylic and/or vinyl monomers or by processing acrylic and/or vinyl polymers and/or copolymers with one or several polar solvents using static or dynamic methods, or by grafting acrylic and/or vinyl monomers on substrates with a glycol polyalkylene base, characterised in that the active acid sites of the said polymer and/or copolymer are totally or partially neutralised by at least one neutralising agent containing the magnesium ion, such that the percentage of neutralisation of the active acid sites by the magnesium ion varies between 35% and 90% and, in addition, possibly by at least one neutralising agent having a monovalent ion capable of neutralising active acid sites by up to 65%, and in that the degree of bio-degradability is greater than 50% at the end of 36 days as measured by the modified Sturm test.

2. Water-soluble, bio-degradable acrylic and/or vinyl polymer and/or copolymer as claimed in claim 1, characterised in that the active acid sites of the said polymer and/or copolymer are totally or partially neutralised by at least one neutralising agent containing the magnesium ion, such that the percentage of neutralisation of the active acid sites by the magnesium ion varies between 35% and 60% and, in addition, possibly by at least one neutralising agent having a monovalent ion that is capable of neutralising active acid sites by up to 65% and in that the degree of bio-degradability is greater than 50% at the end of 36 days as measured by the modified Sturm test.

3. Water-soluble, bio-degradable acrylic or vinyl polymer and/or copolymer as claimed in claim 1, characterised in that the active acid sites of the said polymer and/or copolymer are totally or partially neutralised by at least one neutralising agent containing the magnesium ion, such that the percentage of neutralisation of the active acid sites by the magnesium ion varies between 60% and 90% and, in addition, possibly by at least one neutralising agent having a monovalent ion capable of neutralising the active acid sites by up to 40% and in that the degree of bio-degradability is greater than 50% at the end of 36 days as measured by the modified Sturm test.

4. Water-soluble, bio-degradable acrylic and/or vinyl polymer and/or copolymer as claimed in one of claims 1 to 3, characterised in that the monovalent ion of the neutralising agent is selected from the sodium, potassium, lithium, ammonium ions or from the primary, secondary or tertiary aliphatic and/or cyclic amines.

5. Water-soluble, bio-degradable acrylic and/or vinyl polymer and/or copolymer as claimed in one of claims 1, 2 or 4, characterised in that 50% of the active acid sites of the polymer and/or copolymer are neutralised by a neutralising agent containing the magnesium ion and 50% by a neutralising agent containing the sodium ion.

6. Water-soluble, bio-degradable acrylic and/or vinyl polymer and/or copolymer as claimed in claims 1, 3 or 4, characterised in that 75% of the active acid sites of the said polymer and/or copolymer are neutralised by a neutralising agent containing the magnesium ion and 25% by a neutralising agent containing the sodium ion.

7. Water-soluble, bio-degradable acrylic and/or vinyl polymer and/or copolymer as claimed in one of claims 1, 3 or 4, characterised in that 88% of the active acid sites of the said polymer and/or copolymer are neutralised by a neutralising agent containing the magnesium ion and 12% by a neutralising agent containing the sodium ion.

8. Water-soluble, bio-degradable acrylic and/or vinyl polymer and/or copolymer as claimed in any one of claims 1 to 7, characterised in that it has a specific viscosity of not more than 25 and preferably not more than 10.

9. Water-soluble, bio-degradable acrylic and/or vinyl polymer and/or copolymer as claimed in claim 8, characterised in that it is a solution.

10. Water-soluble, bio-degradable acrylic and/or vinyl polymer and/or copolymer as claimed in claim 8, characterised in that it is a powder.

11. Application of the water-soluble, bio-degradable acrylic and/or vinyl polymer and/or copolymer as claimed in claims 1 to 10 to the fields of paper, paints, plastics, petroleum, drilling fluids, water treatment, detergents, cosmetics, textiles, inks, leather and the crushing and/or breaking up of minerals in water.

12. Method of imparting to water-soluble polymers and/or copolymers a degree of bio-degradability in excess of 50% at the end of 36 days as measured by the modified Sturm test, characterised in that it involves a stage of total or partial neutralisation of the active acid sites of the said polymers and/or copolymers by an agent containing the magnesium ion.

13. Water-soluble polymer and/or copolymer having a degree of bio-degradability in excess of 50% at the end of 36 days as measured by the modified Sturm test, obtained by the method as claimed in claim 12.